# EUROPÄISCHE PATENTSCHRIFT

(11) **EP 2 675 780 B1**
(45) Veröffentlichungstag und Bekanntmachung des Hinweises auf die Patenterteilung: **01.10.2014**
(21) Anmeldenummer: 12703321.5
(22) Anmeldetag: 13.02.2012
(51) Int. Cl.: C07C 41/08, C07C 43/16

(54) **VERFAHREN ZUR HERSTELLUNG VON DIVINYLETHERN**
PROCESS FOR PREPARING DIVINYL ETHERS
PROCÉDÉ DE PRODUCTION D'ÉTHERS DIVINYLIQUES

(30) Priorität: 17.02.2011 EP 11154778
(43) Veröffentlichungstag der Anmeldung: 25.12.2013
(73) Patentinhaber: BASF SE, 67056 Ludwigshafen (DE)
(72) Erfinder: MAIDHOF, Thomas, 55437 Ockenheim (DE); KRONEMAYER, Helmut, 69121 Heidelberg (DE); GROSCH, Georg, Heinrich, 67098 Bad Dürkheim (DE); VOGELSANG, Regina, 67061 Ludwigshafen (DE); GUMBEL, Klaus, Peter, 68165 Mannheim (DE); BALENSIEFER, Tim, 68161 Mannheim (DE); SHILKIN, Alexey, 67071 Ludwigshafen (DE)
(86) Internationale Anmeldenummer: PCT/EP2012/052369
(87) Internationale Veröffentlichungsnummer: WO 2012/110437

(56) Entgegenhaltungen:
- EP-A1- 0 906 899
- SHOSTAKOVSKII M F ET AL: "Vinyl ethers of di- and tri- ethylene glycols", JOURNAL OF GENERAL CHEMISTRY USSR, CONSULTANTS BUREAU, NEW YORK, NY, US, Nr. 7, 1. Juli 1964 (1964-07-01), Seiten 2125-2128, XP009159135, ISSN: 0022-1279

## Beschreibung

Die Erfindung betrifft ein Verfahren zur Herstellung von Divinylethern durch Umsetzung von Verbindungen mit zwei Hydroxylgruppen (im Nachfolgenden Diole genannt) mit Acetylen, welches dadurch gekennzeichnet ist, dass
- keine vollständige Umsetzung der Hydroxylgruppen mit Acetylen erfolgt und das erhaltene Produktgemisch neben dem Divinylether daher auch noch den Monovinylether enthält und
- der Monovinylether durch eine Extraktivdestillation in Gegenwart eines Extraktionsmittels aus dem Produktgemisch abgetrennt wird.

Vinylether, auch Divinylether, können nach dem Reppe-Verfahren hergestellt werden. Eine ausführliche Beschreibung dieses Verfahrens findet sich in Ullmann's Encyclopedia of Industrial Chemistry, Vol. 38, Seiten 77 bis 85. Das wesentliche Merkmal dieses Verfahrens ist die Umsetzung von Hydroxyverbindungen mit Acetylen zu den entsprechenden Vinylethern. Die Umsetzung erfolgt im Allgemeinen in Gegenwart einer Alkalibase, z.B. das Alkoholat der Hydroxyverbindungen, welches durch Zugabe von Alkalihydroxid zur Hydroxyverbindung gebildet wird.

Bei Verbindungen mit zwei Hydroxygruppen (Diolen) bildet sich im Allgemeinen zunächst der Monovinylether des Diols; erst danach setzt sich weiteres Acetylen mit der zweiten Hydroxygruppe zum Divinylether um. Für eine vollständige Umsetzung zum Divinylether werden lange Reaktionszeiten benötigt, da mit abnehmender Zahl der noch vorhandenen Hydroxygruppen die Reaktionswahrscheinlichkeit immer geringer wird. Auch geht mit der Umsetzung eine Viskositätszunahme einher, die einen vollständigen Umsatz zum Divinylether erschwert. Das Problem der Viskositätszunahme tritt in besonderer Weise bei der Umsetzung des Diols mit Acetylen in Abwesenheit einer zusammenhängenden Gasphase auf. Eine derartige Umsetzung mit Acetylen ist aus EP-A 733401 bekannt. Dabei wird der Reaktor vollständig gefüllt und das gasförmige Acetylen in die flüssige Phase einleitet, so dass es sich in der flüssigen Phase verteilt, ohne dass es zur Ausbildung einer zusammenhängenden Gasphase des Acetylens kommt. Bei diskontinuierlicher Verfahrensweise wird der Reaktorinhalt in einen Ausgleichbehälter entspannt und das Reaktionsgemisch in den Reaktor zurückgeführt. Bei kontinuierlicher Verfahrensweise wird der Reaktorinhalt ebenfalls in einen Ausgleichsbehälter entspannt; dieser wird aber nicht wieder in den Reaktor zurückgeführt sondern kontinuierlich der nachgelagerten Aufarbeitung zugeführt. Diese Verfahrensweisen sind in besonderer Weise durch hohe Viskositäten des Reaktionsgemisches erschwert.

Aus EP-A 906 899 ist daher bekannt, die Umsetzung abzubrechen, bevor ein vollständiger Umsatz zum Divinylether erreicht ist. Aus dem erhaltenen Produktgemisch wird Monovinylether destillativ nach Zusatz von Metallhydroxid abgetrennt. Nachteilig an diesem Verfahren ist, dass der Zusatz von Metallhydroxid nur bei Monovinylethergehalten von 0 - 3 % technisch machbar und sinnvoll ist. Bei Monovinylethergehalten größer 3 % führen die Monovinylether-Metallsalze zu hohen Viskositäten und Verstopfungen in den Kolonnensümpfen.

Aufgabe der vorliegenden Erfindung war ein möglichst einfaches kontinuierlich durchführbares Verfahren zur Herstellung von Divinylethern in hoher Reinheit und Ausbeute. Insbesondere ist ein Verfahren gewünscht bei dem keine zusätzlichen Einsatzstoffe benötigt werden.

Demgemäß wurde das eingangs definierte Verfahren gefunden.

Bei dem erfindungsgemäßen Verfahren werden Verbindungen mit zwei Hydroxylgruppen (Diole) mit Acetylen umgesetzt.

Bei den Diolen handelt es sich vorzugsweise um aliphatische oder cycloaliphatische Verbindungen mit einem Molekulargewicht kleiner 500 g/mol, insbesondere kleiner 300 g/mol. Vorzugsweise enthalten die Diole außer den beiden Hydroxylgruppen keine weiteren funktionellen Gruppen oder Heteroatome.

Bevorzugte Diole sind solche der Formel I

HO-X-OH

oder der Formel II 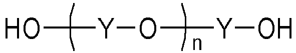 worin X und Y für eine C2- bis C10-Alkylengruppe stehen und n eine ganze Zahl von 1 bis 10 bedeutet.

Besonders bevorzugt steht X in Formel I für eine C2- bis C6-Alkylengruppe. Besonders bevorzugte Verbindungen der Formel I sind 1,2- Dihydroxyethan (Ethylenglykol), 1,3-Dihydroxypropan, 1,4-Dihydroxybutan oder 1,6 Dihydroxyhexan.

Besonders bevorzugt steht Y in Formel II für eine C2- bis C4-Alkylengruppe. n steht besonders bevorzugt für eine ganze Zahl von 1 bis 6, insbesondere steht n für 1, 2, 3 oder 4.

In einer besonders bevorzugten Ausführungsform steht Y für eine Ethylengruppe und n für eine ganze Zahl von 1 bis 4.

Als besonders bevorzugte Diole für das erfindungsgemäße Verfahren seien die Verbindungen Diethylenglykol (n=1) und Triethylenglycol (n=2) der Formel II genannt.

In Betracht kommt auch, Gemische von Diolen einzusetzen, die nachstehenden Ausführungen gelten daher auch für Gemische; bevorzugt handelt es sich aber um ein reine Diole, die allenfalls übliche technische Verunreinigungen enthalten können.

Die Umsetzung des Diols mit Acetylen erfolgt vorzugsweise in Gegenwart eines Katalysators. Bevorzugt wird das Alkalialkoholat des Diols als Katalysator verwendet. Dazu wird das Diol vor der Umsetzung mit Acetylen durch Zusatz von Natriumhydroxid oder Kaliumhydroxid in fester oder wässriger Form teilweise in das entsprechende Natrium- oder Kaliumalkoholat überführt. Vorzugsweise werden 0,1 bis 20, insbesondere 0,5 bis 10 mol % der Hydroxygruppen in Alkoholatgruppen überführt und die Menge Alkalihydroxyid entsprechend gewählt.

Die vorstehende Umsetzung des Diols mit Alkalihydroxyid kann diskontinuierlich oder kontinuierlich durchgeführt werden. Während der Umsetzung wird das Gemisch vorzugsweise entwässert. Eine diskontinuierliche Durchführung erfolgt vorzugsweise unter vermindertem Druck und Temperaturen bis zu 200°C, insbesondere bei 1 bis 100 mbar und 140 bis 180°C. Kontinuierlich kann die Umsetzung mit dem Alkalihydroxid als Reaktivdestillation mit destillativer Abtrennung des Wassers durchgeführt werden.

Bevorzugt wird für die Umsetzung mit Acetylen ein Diol mit dem oben angegebenen Gehalt an Alkoholatgruppen und einem Wassergehalt kleiner 1 Gew. %, insbesondere kleiner 0,5 Gew. % verwendet.

Die Umsetzung des Acetylens mit dem Diol erfolgt vorzugsweise bei Temperaturen von 80 bis 220, besonders bevorzugt bei 140 bis 180°C und vorzugsweise bei Drucken von 1,0 (Normaldruck) bis 50 bar und besonders bevorzugt von 5 bis 30 bar.

Die Umsetzung kann in beliebigen Reaktoren erfolgen, genannt seien z.B. Rührkessel, Rührkesselkaskaden, Rohrreaktoren oder Schlaufenreaktoren. Vorzugsweise erfolgt die Umsetzung in einem Schlaufenreaktor.

Insbesondere erfolgt die Umsetzung unter Vermeidung einer zusammenhängenden Gasphase des Acetylen, wie auch in EP-A 733401 beschrieben ist. Dazu wird das Reaktionsgefäß vorzugsweise mit dem Diol vollständig gefüllt und Acetylen in die flüssige Phase bis zum gewünschten Sättigungsgrad eingeführt. Ein Ausgangstrom wird vorzugsweise in einen Ausgleichsbehälter entspannt und in den Reaktor zurückgeführt um so die Zusammensetzung und Acetylenkonzentration im Reaktionsgemisch zu bestimmen. Die Erfindung ist aber darüber hinaus in besonderer Weise auch für eine kontinuierliche Verfahrensweise geeignet. Bei kontinuierlicher Verfahrensweise wird der Reaktorinhalt nach Entspannung in ein Ausgleichsgefäß, vorzugsweise nicht wieder in den Reaktor zurückgeführt, sondern kontinuierlich der nachgelagerten Aufarbeitung zugeführt.

Erfindungsgemäß erfolgt keine vollständige Umsetzung zum Divinylether. Die Umsetzung wird nur soweit geführt, dass noch die gewünschte Menge an Monovinylether im Produktgemisch enthalten ist. Vorzugsweise enthält das Produktgemisch
30 bis 98 Gew. % Divinylether und
2 bis 70 Gew. % Monovinylether,
bezogen auf die Gewichtssumme aus Divinylether und Monovinylether.

### Besonders bevorzugt enthält das Produktgemisch

60 bis 95 Gew. % Divinylether und
5 bis 40 Gew. % Monovinylether,
bezogen auf die Gewichtssumme aus Divinylether und Monovinylether.

Weiterhin kann das Produktgemisch auch nicht umgesetztes Diol, bei dem keine der Hydroxygruppen in Vinylethergruppen überführt sind, enthalten, z.B. in Mengen von 0 bis 40, insbesondere 0,5 bis 30 Gewichtsteilen auf 100 Gewichtsteilen der Gewichtssumme von Divinylether und Monovinylether. Im Übrigen enthält das Produktgemisch basische Salze der Alkalimetalle als Nebenprodukte bzw. als nicht umgesetzte Ausgangsstoffe.

Bei einer anschließenden Aufarbeitung des Produktgemisches können zunächst die basischen Salze der Alkalimetalle abgetrennt werden. Dies kann z. B in einem Dünnschichtverdampfer erfolgen, durch den das gesamte Produktgemisch oder nur ein Teilstrom des Produktgemisches geführt werden.

Wesentliches Merkmal des erfindungsgemäßen Verfahrens ist die Entfernung des Monovinylethers aus dem Produktgemisch durch eine Extraktivdestillation.

Die Extraktivdestillation kann sich an eine vorherige Aufarbeitung, z.B. die vorstehende Salzabtrennung, anschließen.

Es ist ein Vorteil des erfindungsgemäßen Verfahrens, dass für die Extraktivdestillation das Produktgemisch direkt aus der Umsetzung ohne sonstige Aufarbeitung bzw. ohne vorherige Abtrennung von Nebenprodukten, Katalysatoren (Salzabtrennung) oder nicht umgesetzten Ausgangsstoffen verwendet werden kann.

Das Produktgemisch wird daher vorzugsweise aus dem Reaktor direkt der Extraktiv-destillation zugeführt.

Als Extraktionsmittel geeignet sind organische Lösemittel, in denen der Monovinylether ausreichend löslich ist. In Betracht kommen insbesondere aliphatische und cycloaliphatische Verbindungen mit einem Molgewicht unter 200 g/mol. Insbesondere geeignet sind aliphatische oder cycloaliphatische Verbindungen mit mindestens einer Hydroxygruppe oder Carbonylgruppe.

Insbesondere sind die obigen Diole der Formel I und II als Extraktionsmittel geeignet.

In einer besonders bevorzugten Ausführungsform werden die bei der Umsetzung mit Acetylen eingesetzten Diole auch als Extraktionsmittel verwendet, um Monovinylether dieser Diole aus dem Produktgemisch abzutrennen.

Entsprechend wird vorzugsweise Diethylenglykol

HO-CH₂-CH₂-O-CH₂-CH₂-OH

bei der Herstellung des Diethylenglykoldivinyethers

H₂C=CH-O-CH₂-CH₂-O-CH₂-CH₂-O-CH=CH₂

und vorzugsweise Triethylenglykol

HO-CH₂-CH₂-O-CH₂-CH₂-O-CH₂-CH₂-OH

bei der Herstellung des Triethylenglykoldivinylethers

H₂C=CH-O-CH₂-CH₂-O-CH₂-CH₂-O-CH₂-CH₂-O-CH =CH₂

als Extraktionsmittel verwendet.

Das Extraktionsmittel wird vorzugsweise in einer Menge von 5 bis 100 Gewichtsteilen, besonders bevorzugt von 20 bis 80 Gewichtsteilen, insbesondere 30 bis 60 Gewichtsteilen auf 100 Gewichtsteile der Gewichtssumme von Mono- und Divinylether verwendet. Bei kontinuierlicher fahrweise entsprechen diese Gewichtsteile dem Gewichtsverhältnis der zugeführten Ströme.

Die Extraktivdestillation wird insbesondere bei 60 bis 240°C und bei einem vermindertem Druck von 0,001 bis 1 bar durchgeführt. Zum Beispiel liegt bei einem verminderten Druck von 1 bis 50 mbar eine geeignete Sumpftemperatur bei 130 bis 190°C und Kopftemperatur bei 80 bis 120°C.

Im Sumpf der Kolonne wird ein Gemisch erhalten, in dem der Monovinylether angereichert ist (Sumpfgemisch).

Oberhalb des Sumpfes kann an geeigneter Stelle ein Gemisch entnommen werden, in dem der gewünschte Divinylether angereichert ist (Produktgemisch).

Aus dem Sumpfgemisch kann der Monovinylether abgetrennt und in die Umsetzung mit Acetylen zurückgeführt werden. Wenn das bei der Umsetzung verwendete Diol auch gleichzeitig das Extraktionsmittel ist (siehe obige bevorzugte Ausführungsformen), kann eine Aufarbeitung des mit Monovinylether angereicherten Sumpfgemisches entfallen und das gesamte Sumpfgemisch kann ohne Aufarbeitung zurückgeführt werden, sei es in die Umsetzung mit dem Alkalihydroxid (siehe oben) oder direkt in die Umsetzung mit dem Acetylen; in diesem Fall wird die Menge des Extraktionsmittel (das heißt des entsprechenden Diols) naturgemäß vorzugsweise so gewählt, dass das Volumen des erhaltenen Sumpfgemisches nicht das zur Verfügung stehende Volumen des Reaktors, in den zurückgeführt wird, übersteigt; besonders bevorzugt wird die Menge des Extraktionsmittels so gewählt, dass das Volumen des erhaltenen und zurückgeführten Sumpfgemisches dem zur Verfügung stehenden Volumen des Reaktors, in den zurückgeführt wird, entspricht und dieses vollständig ausnutzt

Je nach apparativer Ausgestaltung, insbesondere je nach Anzahl der Böden, kann bei der Extraktivdestillation direkt ein Produktgemisch erhalten werden, welches aus dem Divinylether in der gewünschten Reinheit besteht. Alternativ kann bei weniger aufwendigen apparativen Auslegung ein Produktgemisch entnommen werden, in dem der Divinylether angereichert ist, aber auch noch Diol und gegebenenfalls abgereicherte Mengen Monovinylether enthalten sind. Dieses Produktgemisch kann dann weiter destillativ aufgetrennt werden, so dass der Divinylether in der gewünschten Reinheit erhalten wird. Das dabei anfallende Diol und ggf. der restliche Monovinylether können wiederum ohne Aufarbeitung wie vorstehend beschrieben zurückgeführt werden, wenn das Extraktionsmittel mit dem bei der Umsetzung verwendeten Diol identisch ist.

Geeignete Kolonnen für die Extraktivdestillation sind Füllkörperkolonnen oder Packungskolonnen, welche insbesondere kontinuierlich betrieben werden.

Geeignet sind auch Kolonnen, die sowohl Füllkörper als auch Packungen enthalten, z.B. im unteren Teil Füllkörperschüttungen enthalten und im oberen Teil mit Packungselementen (z.B. eingebaute Stahlbleche) aufweisen.

Die Kolonnen können vorzugsweise mindestens zwei, besonders bevorzugt mindestens 3 theoretische Böden haben. Die Anzahl der theoretischen Böden kann z.B. 2 bis 100, insbesondere 3 bis 20 betragen.

Bei einer Extraktivdestillation kann z.B.
ein Sumpfgemisch aus
10 bis 70 Gew.%, insbesondere 15 bis 50 Gew. % Monovinylether und besonders bevorzugt 20 bis 40 Gew. % Monovinylether
2 bis 50 Gew.%, insbesondere 2 bis 40 Gew.% Divinylether- und besonders bevorzugt 5 bis 30 Gew.% Divinylether ,
10 bis 90 Gew. %, insbesondere 30 bis 90 Gew. % Diol und und besonders bevorzugt 40 bis 80 Gew. % Diol
0 bis 20 Gew. %, insbesondere 0 bis 10 Gew. % sonstigen Bestandteilen
und ein Produktgemisch aus
0 bis 15 Gew. %, insbesondere 0 bis 5 Gew. % Monovinylether, besonders bevorzugt 0 bis 2 Gew.% Monovinylether
60 bis 99,5 Gew. %, insbesondere 80 bis 99,5 Gew. % Divinylether, besonders bevorzugt 90 bis 99,5 Divinylether
0,2 bis 20 Gew. %, insbesondere 0,2 bis 10 Gew. % Diol und besonders bevorzugt 0,2 bis 5 Gew. % Diol und
0 bis 10 Gew. %, insbesondere 0 bis 5 Gew. % sonstigen Bestandteilen
erhalten werden.

Aus dem Produktgemisch kann der Divinylether leicht in einer Reinheit von mehr als 99 Gew.%, insbesondere von mehr als 99,5 Gew. %, destillativ abgetrennt werden, wobei der Gehalt an Diol im Divinylether vorzugsweise kleiner 0,5 Gew. % und insbesondere kleiner 0,2 Gew. % ist Der Divinylether kann bei entsprechender apparativer Auslegung mit gleicher Reinheit auch bereits direkt aus der Extraktivdestillation gewonnen werden.

Das erfindungsgemäße Verfahren kann diskontinuierlich oder kontinuierlich durchgeführt werden. Auch können bei entsprechenden apparativen Gegebenheiten kontinuierlich durchgeführte Verfahrensschritte mit diskontinuierlich durchgeführten kombiniert werden. In einer bevorzugten Ausführungsform wird das Verfahren mit allen Verfahrensschritten kontinuierlich durchgeführt.

Das erfindungsgemäße Verfahren eignet sich sowohl für eine diskontinuierliche als auch eine kontinuierliche Verfahrensweise. Es ist technisch einfach durchzuführen. Mit dem Verfahren sind Divinylether in hoher Ausbeute und Reinheit erhältlich. Viskositätszunahmen, welche bei hohen Umsätzen zu Divinylethern auftreten und zu Problemen führen, können vermieden werden.

### Beispiel

### Synthese Diethylenglykoldivinylether

Diethylenglykol reagiert mit Acetylen unter Verwendung von KOH als Base (Bildung des Alkoholats) zum Divinylether bzw. zu einem Gemisch von Divinylether und Monovinylether (bei Teilumsatz) gemäß nachstehender Reaktionsgleichung:

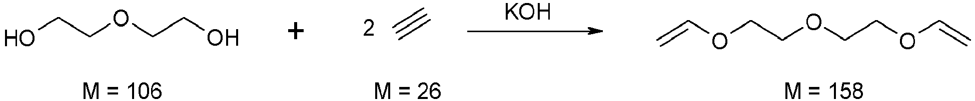

### Abkürzungen:

Diethylenglykol: DEG
Diethylenglykol-monovinylether: DEG-MVE
Diethylenglykol-divinylether: DEG-DVE

### 1. Herstellung des Gemisches aus Mono- und Divinyletherglykol

| | | | |
|---|---|---|---|
| Ansatz: | 1720 g | Diethylenglykol | = 16,2 mol |
| | 280 g | Diethylenglykoldivinylether | = 1,8 mol |
| | 50,0 g | KOH Plätzchen | |

Die Komponenten wurden in einen 2.5 Liter Autoklaven eingefüllt. Acetylen wurde dem Autoklav zugeführt bis der Druck 19 bar betrug. Die Umsetzung erfolgte bei 155°C, 19 bar und einer Drehzahl des Rührers von 700 U/min. Nachdem 550 Liter Acetylen aufgenommen worden sind (etwa nach 7 Stunden) wurde die Reaktion abgebrochen, der Autoklav entspannt und auf Raumtemperatur abgekühlt.

Es wurde ein Gemisch aus Mono- und Divinylether des Ethylenglykol erhalten. Die gaschromatographische Analyse ergab ein Verhältnis von DEG-DVE zu DEG-MVE von 68 zu 29 (Flächenprozent).

### 2. Salzabtrennung

Eine aus einer Reaktion von DEG mit Acetylen erhaltene Rohware (mit ca. 19% DEG-MVE) wird mittels Dünnschichtverdampfer kontinuierlich bei einer Wärmeträgertemperatur im Verdampfer von 110 °C und einem Druck von 10 mbar abs. mit ca. 300 ml/h und Zugabe von ca. 10% inerten Sumpfverdünner (Pluriol® E600) von den basischen Salzen befreit. Das erhaltene Destillat enthält ca. 14 Gew. % DEG-MVE.

### 3. Extraktivdestillation

Mit einem Zulaufstrom von ca. 300 g/h wird das Rohdestillat (mit 14% DEG-MVE) auf den unteren Schuss einer entsprechend dimensionierten Destillationskolonne mit Kopf-, Sumpf- und Seitenabzug und strukturierter Packung (Sulzer DX, Packungshöhe ca. 0,95 m) gefahren. Es wird ein Rücklaufverhältnis von 20:1 am Kopf und 1:1 am Seitenabzug eingestellt.
Die Sumpftemperatur betrug 105°C.
Bei einem Vakuum von ca. 20 mbar stellt sich eine Kopftemperatur von ca. 74°C ein. Gleichzeitig wird in Höhe des Seitenabzuges ein Strom von ca. 140 g/h DEG als Extraktionsmittel zugefahren, wodurch man eine Anreicherung von DEG-MVE im Sumpf der Kolonne erreicht (ca. 30 % DEG-MVE, 60 % DEG und 10 % DEG-DVE).
Der Strom aus dem Sumpf der Kolonne (ca. 204 g/h) kann nun abgezogen und wieder der Basenpräparation oder der Umsetzung zugeführt werden.
Am flüssigen Seitenabzug der Kolonne werden ungefähr 216 g/h abgezogen (Zusammensetzung ca. 0,1 % DEG-MVE, 1 % DEG und 99 % DEG-DVE) und der Reindestillation zugeführt. Am Kopf der Kolonne werden ca. 15 g/h Leichtsieder abgezogen.

### 4. Reindestillation (batch/konti)

Der Strom aus dem Seitenabzug der Extraktivdestillation muss nun in der Reindestillation noch von DEG befreit werden. Dazu wird er mit ca. 221 g/h in den Sumpf einer weiteren Destillationskolonne (einfache Kopf- Sumpf- Trennung ohne Seitenabzug) mit strukturierter Packung gefahren.
Die Sumpftemperatur sollte dabei bei etwa 112°C liegen.
Bei einem Vakuum von ca. 20 mbar stellt sich eine Kopftemperatur von ca. 85°C ein.
In der Kolonne wird am Kopf ein Rücklaufverhältnis von 3:1 eingestellt.
Am Kopf der Kolonne werden ungefähr 209 g/h Wertprodukt mit einer Reinheit von min. 99 % (und ca. 0,1% DEG) abgezogen
Aus dem Sumpf der Kolonne wird ein Strom von etwa 12 g/h abgezogen, der wieder der Basenpräparation oder der Umsetzung zugeführt werden kann.

### 5. Rückführung des Sumpfes aus der Extraktivdestillation und erneute Umsetzung

1596 g eines Sumpf aus der Extraktivdestillation wurden erneut mit Acetylen umgesetzt. Der Sumpf hatte folgende Zusammensetzung:

| | | |
|---|---|---|
| 3,9 % | Diethylenglykol-divinylether | |
| 50,6 % | Diethylenglykol-monovinylether | (6,1 mol) |
| 43,5 % | Diethylenglykol | (6,5 mol) |

Kaliumgehalt laut Analytik (Elementaranalyse): 1,5%

Die Umsetzung erfolgte wiederum in einem 2,5 Liter- Autoklaven wie oben beschrieben.

Die gaschromatographische Analyse ergab 94,5 Flächenprozent DEG-DVE. Ein Gehalt an DEG-MVE konnte nicht mehr bestimmt werden.

## Patentansprüche

1. Verfahren zur Herstellung von Divinylethern durch Umsetzung von Verbindungen mit zwei Hydroxylgruppen (im Nachfolgenden Diole genannt) mit Acetylen, **dadurch gekennzeichnet, dass**
- keine vollständige Umsetzung der Hydroxylgruppen mit Acetylen erfolgt und das erhaltene Produktgemisch neben dem Divinylether daher auch noch den Monovinylether enthält und
- der Monovinylether durch eine Extraktivdestillation in Gegenwart eines Extraktionsmittels aus dem Produktgemisch abgetrennt wird.

2. Verfahren gemäß Anspruch 1, **dadurch gekennzeichnet, dass** es sich bei den Diolen um aliphatische oder cycloaliphatische Verbindungen mit einem Molekulargewicht kleiner 500 g/mol handelt.

3. Verfahren gemäß Anspruch 1 oder 2, **dadurch gekennzeichnet, dass** es sich bei den Diolen um aliphatische Diole der Formel I
HO-X-OH
oder der Formel II 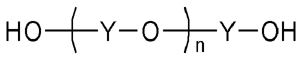 worin X und Y für eine C2- bis C10-Alkylengruppe stehen und n eine ganze Zahl von 1 bis 10 bedeutet,
handelt.

4. Verfahren gemäß einem der Ansprüche 1 bis 3, **dadurch gekennzeichnet, dass** es sich bei dem Diol um Diethylenglykol oder Triethylenglycol handelt.

5. Verfahren gemäß einem der Ansprüche 1 bis 4, **dadurch gekennzeichnet, dass** das erhaltene Produktgemisch 30 bis 95 Gew. % Divinylether und 5 bis 70 Gew. % Monovinylether, bezogen auf die Gewichtssumme aus Divinylether und Monovinylether, enthält.

6. Verfahren gemäß einem der Ansprüche 1 bis 5, **dadurch gekennzeichnet, dass** die Umsetzung des Diols mit Acetylen in Abwesenheit einer zusammenhängenden Gasphase erfolgt.

7. Verfahren gemäß einem der Ansprüche 1 bis 6, **dadurch gekennzeichnet, dass** das gesamte Produktgemisch der Extraktivdestillation zugeführt wird, ohne dass eine vorherige Abtrennung von Nebenprodukten, Katalysatoren oder nicht umgesetzten Ausgangsstoffen erfolgt.

8. Verfahren gemäß einem der Ansprüche 1 bis 7, **dadurch gekennzeichnet, dass** das Extraktionsmittel in einer Menge von 5 bis 100 Gewichtsteilen auf 100 Gewichtsteile Monound Divinylether verwendet wird.

9. Verfahren gemäß einem der Ansprüche 1 bis 8, **dadurch gekennzeichnet, dass** die Extraktion bei 60 bis 240°C und 0,001 bis 1 bar durchgeführt wird.

10. Verfahren gemäß einem der Ansprüche 1 bis 9, **dadurch gekennzeichnet, dass** der bei der Extraktiv-destillation abgetrennte Monovinylether in die Umsetzung zurückgeführt wird.

11. Verfahren gemäß einem der Ansprüche 1 bis 10, **dadurch gekennzeichnet, dass** es sich bei dem Extraktionsmittel um das bei der Umsetzung verwendete Diol handelt.

12. Verfahren gemäß Anspruch 11, **dadurch gekennzeichnet, dass** der abgetrennte Monovinylether im Gemisch mit Diol in die Umsetzung zurückgeführt wird.

13. Verfahren gemäß einem der Ansprüche 1 bis 12, **dadurch gekennzeichnet, dass** es sich um ein kontinuierliches Verfahren handelt.

## Claims

1. A process for preparing divinyl ethers by reacting compounds having two hydroxyl groups (hereinafter referred to as diols) with acetylene, wherein
- the hydroxyl groups are incompletely reacted with acetylene and the resulting product mixture therefore comprises the monovinyl ether in addition to the divinyl ether and
- the monovinyl ether is separated off from the product mixture by extractive distillation in the presence of an extractant.

2. The process according to claim 1, wherein the diols are aliphatic or cycloaliphatic compounds having a molecular weight of less than 500 g/mol.

3. The process according to claim 1 or 2, wherein the diols are aliphatic diols of the formula I
HO-X-OH
or of the formula II 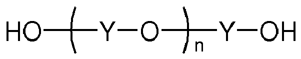 where X and Y are each a C2-C10-alkylene group and n is an integer from 1 to 10.

4. The process according to any of claims 1 to 3, wherein the diol is diethylene glycol or triethylene glycol.

5. The process according to any of claims 1 to 4, wherein the product mixture obtained comprises from 30 to 95% by weight of divinyl ether and from 5 to 70% by weight of monovinyl ether, based on the total weight of divinyl ether and monovinyl ether.

6. The process according to any of claims 1 to 5, wherein the reaction of the diol with acetylene is carried out in the absence of a contiguous gas phase.

7. The process according to any of claims 1 to 6, wherein the entire product mixture is fed to the extractive distillation without a prior removal of by-products, catalysts or unreacted starting materials being carried out.

8. The process according to any of claims 1 to 7, wherein the extractant is used in an amount of from 5 to 100 parts by weight per 100 parts by weight of monovinyl and divinyl ethers.

9. The process according to any of claims 1 to 8, wherein the extraction is carried out at from 60 to 240°C and from 0.001 to 1 bar.

10. The process according to any of claims 1 to 9, wherein the monovinyl ether separated off in the extractive distillation is returned to the reaction.

11. The process according to any of claims 1 to 10, wherein the extractant is the diol used in the reaction.

12. The process according to claim 11, wherein the monovinyl ether which has been separated off is returned in a mixture with diol to the reaction.

13. The process according to any of claims 1 to 12, wherein the process is a continuous process.

## Revendications

1. Procédé de fabrication d'éthers divinyliques par mise en réaction de composés contenant deux groupes hydroxyle (par la suite nommés diols) avec de l'acétylène, **caractérisé en ce que**
- une réaction totale des groupes hydroxyle avec l'acétylène n'a pas lieu et le mélange de produits obtenu contient par conséquent, en plus de l'éther divinylique, également l'éther monovinylique, et
- l'éther monovinylique est séparé du mélange de produits par une distillation par extraction en présence d'un agent d'extraction.

2. Procédé selon la revendication 1, **caractérisé en ce que** les diols consistent en des composés aliphatiques ou cycloaliphatiques ayant un poids moléculaire inférieur à 500 g/mol.

3. Procédé selon la revendication 1 ou 2, **caractérisé en ce que** les diols consistent en des diols aliphatiques de formule I
HO-X-OH
ou de formule II 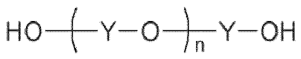 dans lesquelles X et Y représentent un groupe alkylène en C2 à C10, et n signifie un nombre entier de 1 à 10.

4. Procédé selon l'une quelconque des revendications 1 à 3, **caractérisé en ce que** le diol consiste en le diéthylène glycol ou le triéthylène glycol.

5. Procédé selon l'une quelconque des revendications 1 à 4, **caractérisé en ce que** le mélange de produits obtenu contient 30 à 95 % en poids d'éther divinylique et 5 à 70 % en poids d'éther monovinylique, par rapport à la somme en poids de l'éther divinylique et de l'éther monovinylique.

6. Procédé selon l'une quelconque des revendications 1 à 5, **caractérisé en ce que** la mise en réaction du diol avec l'acétylène a lieu en l'absence d'une phase gazeuse connexe.

7. Procédé selon l'une quelconque des revendications 1 à 6, **caractérisé en ce que** l'ensemble du mélange de produits est introduit dans la distillation par extraction, sans qu'une séparation préalable des produits secondaires, des catalyseurs ou des matières premières non réagies n'ait lieu.

8. Procédé selon l'une quelconque des revendications 1 à 7, **caractérisé en ce que** l'agent d'extraction est utilisé en une quantité de 5 à 100 parties en poids pour 100 parties en poids d'éther mono- et divinylique.

9. Procédé selon l'une quelconque des revendications 1 à 8, **caractérisé en ce que** l'extraction est réalisée à 60 à 240 °C et 0,001 à 1 bar.

10. Procédé selon l'une quelconque des revendications 1 à 9, **caractérisé en ce que** l'éther monovinylique séparé lors de la distillation par extraction est recyclé dans la réaction.

11. Procédé selon l'une quelconque des revendications 1 à 10, **caractérisé en ce que** l'agent d'extraction consiste en le diol utilisé lors de la réaction.

12. Procédé selon la revendication 11, **caractérisé en ce que** l'éther monovinylique séparé est recyclé dans la réaction en mélange avec le diol.

13. Procédé selon l'une quelconque des revendications 1 à 12, **caractérisé en ce qu'**il s'agit d'un procédé continu.
